# EUROPEAN PATENT SPECIFICATION

(11) **EP 4 394 369 B1**
(45) Date of publication and mention of the grant of the patent: **18.06.2025**
(21) Application number: 23200349.1
(22) Date of filing: 28.09.2023
(51) Int. Cl.: G01N 27/06, G01N 33/50

(54) **METHOD FOR MEASURING THE ANTIOXIDANT ACTIVITY OF A SUBSTANCE OR A MIXTURE OF SUBSTANCES**
VERFAHREN ZUR MESSUNG DER ANTIOXIDATIVEN WIRKUNG EINER SUBSTANZ ODER EINER SUBSTANZMISCHUNG
PROCÉDÉ DE MESURE D'ACTIVITÉ ANTIOXYDANTE D'UNE SUBSTANCE OU D'UN MELANGE DE SUBSTANCES

(30) Priority: 28.12.2022 IT 202200026997
(43) Date of publication of application: 03.07.2024
(73) Proprietor: B.T.S. S.r.l., 00044 Frascati (RM) (IT); Consiglio per la ricerca in agricoltura e l'analisi dell'economia agraria, 00184 Roma (IT)
(72) Inventor: FINOTTI, Enrico, 00184 ROMA (IT); BRANCALEONI, Ugo, 00044 FRASCATI (RM) (IT)
(74) Representative: Studio Torta S.p.A.

(56) References cited:
- CN-A- 114 902 920
- LUCÍA VELA ET AL: "Antioxidant capacity of Spanish honeys and its correlation with polyphenol content and other physicochemical properties", JOURNAL OF THE SCIENCE OF FOOD AND AGRICULTURE, vol. 87, no. 6, 30 April 2007 (2007-04-30), pages 1069 - 1075, XP055612536, ISSN: 0022-5142, DOI: 10.1002/jsfa.2813
- CAMPANELLA L. ET AL: "Biosensors for determination of total and natural antioxidant capacity of red and white wines: comparison with other spectrophotometric and fluorimetric methods", BIOSENSORS AND BIOELECTRONICS, vol. 19, no. 7, 16 September 2003 (2003-09-16), pages 641 - 651, XP093054329, ISSN: 0956-5663, DOI: 10.1016/S0956-5663(03)00276-8

## Description

### CROSS-REFERENCE TO RELATED APPLICATIONS

This patent application claims priority from Italian patent application no. 102022000026997 filed on December 28, 2022.

### TECHNICAL FIELD

The present invention relates to a method which is useful for measuring the antioxidant activity of a substance or a mixture of substances.

### BACKGROUND

To date, in order to measure the antioxidant activity of a substance, optical techniques involving the use of spectrophotometer are known to be used. Such techniques have the drawback of examining either only hydrophilic solutions or only lipophilic solutions. Such a limitation necessarily involves sample preparation as well as obtaining two results related to hydrophilic antioxidant activity and lipophilic antioxidant activity, respectively. The problem lies in the fact that the total antioxidant activity cannot be considered a mere sum of the hydrophilic and lipophilic ones, besides the fact that the preparation undergone by the substance makes the results untrue.

It should also be considered that not all current spectrophotometric methods are applicable to both hydrophilic and lipophilic solutions and, in addition, some spectrophotometric methods are applicable to clear (hydrophilic or lipophilic) solutions only. The latter limitation dictates that in a complex matrix it is required to proceed with a solvent extraction of both the hydrophilic and lipophilic fractions in order to then be able to measure them with the spectrophotometer.

Several measurement methods involving the use of a target substance have been developed over time. Such methods have the drawback of not uniquely defining the antioxidant ability of a substance, but they detect it based on the oxidation of another substance, the target substance.

In other words, these methods analyze the change in electric potential of a target substance subjected to an oxidative attack before and after introducing the substance to be tested, and correlate the detected change to the antioxidant activity of the substance of interest. Therefore, such methods cannot provide a direct evaluation of the antioxidant activity of the substance of interest, with all the drawbacks this entails. It has been scientifically proven that the interaction with other chemical species necessarily affects the antioxidant activity of a substance. In fact, there is no purely summative effect of the antioxidant activity of several interacting substances, but there is a total antioxidant activity which is precisely the result of the interaction of several chemical species.

One of such known methods relates to a method which operates by measuring the redox potential of a target substance in an emulsion system and in the presence of a radical source. The method provides for the measurement to be repeated even in the presence of the substance whose antioxidant activity is to be measured. The comparison of measurements recorded in the absence and presence of the substance of interest provides the information about its antioxidant activity.

Such method, while being able to overcome the problems related to spectrophotometric techniques, noted above, however, has the problem of using a target substance which necessarily interferes with the measurement.

CN114902920 discloses evaluating the antioxidant activity of Rhizoma Radix extract, wherein a first electrical conductivity value of a reference solution comprising a solvent and a free radical generating substance (DPPH) and a second electrical conductivity value of a sample solution comprising said solvent, said free radical generating substance and said extract are detected.

A need was thus felt for a method for measuring the antioxidant activity of a substance whose technical features could overcome the limitations of the spectrophotometric methods while not requiring the use of a target substance.

### SUMMARY

An object of the present invention is a method for evaluating the antioxidant activity of a substance; said method being characterized in that it comprises a step of measuring and comparing the electrical conductivity comprising the operations of:
- detecting a first electrical conductivity value and a first pH value of a first solution comprising a solvent and an active substance in the production of free radicals;
- detecting a second electrical conductivity value and a second pH value of a second solution comprising said solvent, said active substance in the production of free radicals and a substance whose antioxidant activity is to be evaluated;
- calculating a mathematical relationship between said first electrical conductivity value and said second electrical conductivity value;
said first solution and said second solution having the same temperature, the same volume of solvent, the same composition of solvent and the same concentration of active substance in the production of free radicals.

Preferably, said method comprises a step of measuring and comparing the electric potential comprising the operations of:
- detecting a first electric potential value of said first solution;
- detecting a second electric potential value of said second solution;
- calculating a mathematical relationship between said first electric potential value and said second electric potential value.

The calculation of the mathematical relationship between the first value and second value of electric potential is appropriate if the pH values of the first and second solutions are different.

Preferably, said first and said second electric potential values being detected by means of a pH meter.

Preferably, said mathematical relationship is the ratio between said first electrical conductivity value and said second electrical conductivity value and between said first electric potential value and said second electric potential value.

Preferably, said step of measuring and comparing the electrical conductivity is repeated at different known concentrations of the substance whose antioxidant activity is to be evaluated.

Preferably, said solvent is composed of a mixture of a first solvent, in which the active substance in the production of free radicals is dissolved, and a second solvent, in which the substance whose antioxidant activity is to be evaluated is dissolved.

Preferably, said active substance in the production of free radicals is a diazo compound; said first and said second solutions being maintained at a temperature higher than 36°C.

Preferably, said diazo compound is 2,2'-azo-bis (2-aminopropane)dichloride (APAB).

### BRIEF DESCRIPTION OF THE DRAWINGS

For a more thorough understanding of the invention and advantages thereof, reference will now be made to some illustrative and non-limiting examples together with the accompanying figures, in which
- Figure 1 schematically illustrates the reactor used in the examples;
- Figure 2 is a chart where the results obtained for the different substances on which the measurement was performed are compared.

### DESCRIPTION OF EMBODIMENTS

The method according to the present invention was tested on two distinct substances: gallic acid and vanillic acid.

The substances above are known to have antioxidant activity.

Hereinafter, reference will also be made to a comparison between the identified antioxidant activities of the different substances.

### - Reactor -

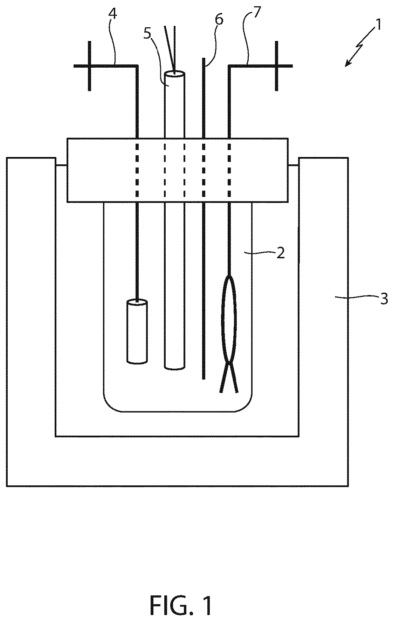

In Figure 1, the reactor used is indicated as a whole with reference number 1. Reactor 1 comprises a reaction tube 2, a rotating assembly 3 adapted to produce the rotation of reaction tube 2 along its own longitudinal axis to promote mixing of the solution, a pH meter 4 adapted to measure pH and, thus, the electric potential of a solution housed within the reaction tube 2, a heating member 5 adapted to heat to a set temperature the solution housed within the reaction tube 2, a temperature meter 6, which measures the temperature of the solution housed within the reaction tube 2 through a thermocouple, and a conductivity meter 7 adapted to measure the electrical conductivity (EC) of a solution housed within a reaction tube 2.

### - Materials -

Each of the test substances (gallic acid, vanillic acid) was dissolved in a methanol stock solution at a concentration of 100 mg/ml.

In addition, 2,2'-azo-bis(2-aminopropane)dichloride (APAB) was used in each of the solutions as an active substance in the production of free radicals at a concentration of 10 mg/ml.

The solvent consists of water, in which APAB is dissolved, and methanol, in which the test substance is dissolved. Regardless of the relative amounts of water and methanol, the total volume should be 40 ml.

### - Measurement -

For each measurement with the test substance (ECS), a respective blank measurement is provided for, where the electrical conductivity is measured without the test substance (EC0). The antioxidant activity is derived from the ECS/ECO ratio.

Each of the blank measurements provided for the same solvent composition as the respective measurement with the test substance. This means that the measurement with the test substance and the respective blank measurement had the same volume of methanol, the difference being that in the measurement with the test substance in the volume of methanol, the test substance was dissolved.

For each of the test substances, the tests were repeated with methanol solution volumes equal to 0.8 ml, 2.0 ml, 4.0 ml, 8.0 ml, 12.0 ml. In this way, measurements at different concentrations of the test substance were performed.

All measurements were performed at a temperature of 40°C. At this temperature, APAB yields free radicals.

### - Results -

Below are two Tables (I and II) each referring to the measurements of one of the two test substances at different concentrations.

The volume of the methanol solution is indicated in the Tables. Such volume for the EC50 measurements will indicate methanol only.

**TABLE I**

| GALLIC ACID | | | | | | |
|---|---|---|---|---|---|---|
| No | Methanol stock solution (µl) | ECS | | | EC0 | ECS/ECO |
| | | Measurement A | Measurement B | Mean value | | |
| 1 | 800 | 5.58 | 5.84 | 5.71 | 2.64 | 2.16 |
| 2 | 2000 | 5.85 | 5.9 | 5.88 | 3.30 | 1.78 |
| 3 | 4000 | 5.38 | 5.33 | 5.36 | 3.2 | 1. 67 |
| 4 | 8000 | 4.46 | 4.33 | 4.40 | 2.97 | 1.48 |
| 5 | 12000 | 4.07 | 4.12 | 4.10 | 3.16 | 1.30 |

**TABLE II**

| VANILLIC ACID | | | | | | |
|---|---|---|---|---|---|---|
| No | Methanol stock solution (µl) | ECS | | | EC0 | ECS/ECO |
| | | Measurement A | Measurement B | Mean value | | |
| 1 | 800 | 6.14 | 6.11 | 6.13 | 4.63 | 1.32 |
| 2 | 2000 | 5.66 | 5.64 | 5.65 | 4.34 | 1.30 |
| 3 | 4000 | 4.88 | 4.73 | 4.81 | 4.06 | 1.18 |
| 4 | 8000 | 4.31 | 4.31 | 4.31 | 3.39 | 1.27 |
| 5 | 12000 | 3.82 | 3.8 | 3.81 | 3.19 | 1.19 |

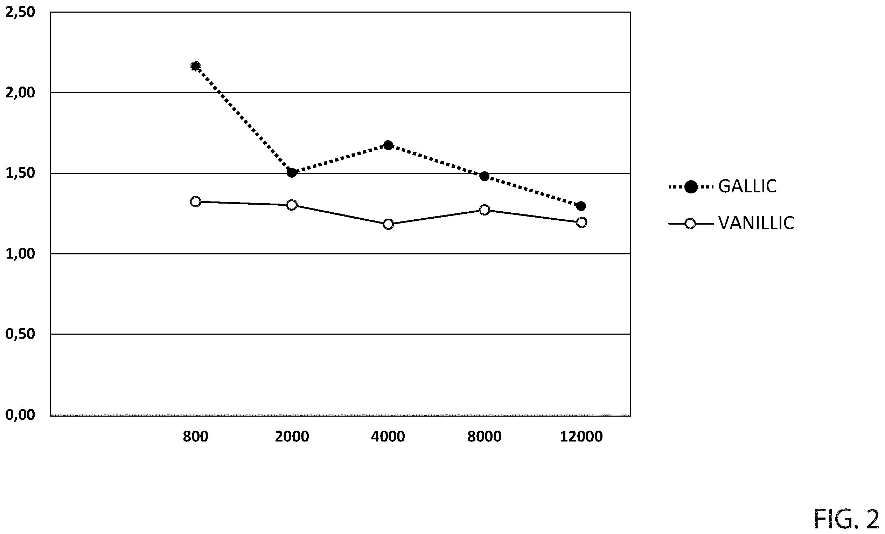

For a comparison about the antioxidant activity, Figure 2 shows the trends of the values obtained for the two test substances in a single chart.

In the chart, the different volumes of methanol stock solution can be found in the x-axis, while the value of the detected ECS/ECO ratio in the y-axis.

The graph in Figure 2 shows that the values change depending on concentration and that, however, the known scale of antioxidant activity of the test substances is respected.

In contrast to the above, and if the pH values related to the solution containing the test substance and the blank solution differ from each other, measurements of the electric potential of the solutions may also be considered but only in addition to, and not in place of, electrical conductivity measurements.

In this regard, it should be specified that for some substances the pH values of the solution are not different depending on the presence or absence of the substance whose antioxidant activity is to be measured.

The subject matter of the present invention is a method allowing to make an antioxidant activity scale of different substances, without having any limitations about the lipophilic or hydrophilic aspect of the solution. In fact, the measurement of electrical conductivity may be carried out in either lipophilic or hydrophilic solvents, which will be chosen based on the type of test substance.

In addition, the absence of a target substance ensures a measurement of the actual antioxidant activity of the test substance, with no possibility of alterations due to interactions with the target substance itself.

## Claims

1. A method for evaluating the antioxidant activity of a substance or a mixture of substances; said method being **characterized in that** it comprises a step of measuring and comparing the electrical conductivity comprising the operations of:
- detecting a first electrical conductivity value and a first pH value of a first solution comprising a solvent and an active substance in the production of free radicals;
- detecting a second electrical conductivity value and a second pH value of a second solution comprising said solvent, said active substance in the production of free radicals and a substance or a mixture of substances whose antioxidant activity is to be evaluated;
**characterised by** calculating a mathematical relationship between said first electrical conductivity value and said second electrical conductivity value;
said first solution and said second solution having the same temperature, the same volume of solvent, the same composition of solvent and the same concentration of active substance in the production of free radicals.

2. The method according to claim 1, **characterized in that** it comprises a step of measuring and comparing the electric potential comprising the operations of:
- detecting a first electric potential value of said first solution;
- detecting a second electric potential value of said second solution;
- calculating a mathematical relationship between said first electric potential value and said second electric potential value.

3. The method according to claim 2, **characterized in that** said first and said second electric potential value being detected by means of a pH meter.

4. The method according to any one of the preceding claims, **characterized in that** said mathematical relationship is the ratio between said first electrical conductivity value and said second electrical conductivity value.

5. The method according to claim 2 or 3, **characterized in that** said mathematical relationship is the ratio between said first electric potential value and said second electric potential value.

6. The method according to one of the preceding claims, **characterized in that** said step of measuring and comparing the electrical conductivity is repeated at different known concentrations of the substance or mixture of substances whose antioxidant activity is to be evaluated.

7. The method according to one of the preceding claims, **characterized in that** said solvent is composed of a mixture of a first solvent, in which the active substance in the production of free radicals is dissolved, and a second solvent, in which the substance or the mixture of substances whose antioxidant activity is to be evaluated is dissolved.

8. The method according to one of the preceding claims, **characterized in that** said active substance in the production of free radicals is a diazo compound; said first and said second solutions being maintained at a temperature higher than 36°C.

9. The method according to claim 8, **characterized in that** said diazo compound is 2,2'-azo-bis (2-aminopropane)dichloride (APAB)

## Patentansprüche

1. Verfahren zur Bewertung der antioxidativen Wirkung einer Substanz oder eines Substanzgemisches, **dadurch gekennzeichnet, dass** es einen Schritt des Messens und des Vergleichens der elektrischen Leitfähigkeit umfasst, umfassend die folgenden Vorgänge:
- Erfassen eines ersten elektrischen Leitfähigkeitswerts und eines ersten pH-Werts einer ersten Lösung, umfassend ein Lösungsmittel und einen Wirkstoff bei der Bildung freier Radikale;
- Erfassen eines zweiten elektrischen Leitfähigkeitswerts und eines zweiten pH-Werts einer zweiten Lösung, umfassend das Lösungsmittel, den Wirkstoff bei der Bildung freier Radikale und eine Substanz oder ein Substanzgemisch, deren antioxidative Wirkung zu bewerten ist;
**gekennzeichnet durch**
Berechnen einer mathematischen Beziehung zwischen dem ersten elektrischen Leitfähigkeitswert und dem zweiten elektrischen Leitfähigkeitswert;
wobei die erste Lösung und die zweite Lösung die gleiche Temperatur, das gleiche Lösungsmittelvolumen, die gleiche Lösungsmittelzusammensetzung und die gleiche Wirkstoffkonzentration bei der Bildung freier Radikale aufweisen.

2. Verfahren nach Anspruch 1, **dadurch gekennzeichnet, dass** es einen Schritt des Messens und Vergleichens des elektrischen Potentials umfasst, umfassend die folgenden Schritte:
- Erfassen eines ersten elektrischen Potentialwerts der ersten Lösung;
- Erfassen eines zweiten elektrischen Potentialwerts der zweiten Lösung;
- Berechnen einer mathematischen Beziehung zwischen dem ersten elektrischen Potentialwert und dem zweiten elektrischen Potentialwert.

3. Verfahren nach Anspruch 2, **dadurch gekennzeichnet, dass** der erste und der zweite elektrische Potentialwert mit Hilfe eines pH-Meters erfasst werden.

4. Verfahren nach einem der vorstehenden Ansprüche, **dadurch gekennzeichnet, dass** die mathematische Beziehung das Verhältnis zwischen dem ersten elektrischen Leitfähigkeitswert und dem zweiten elektrischen Leitfähigkeitswert ist.

5. Verfahren nach Anspruch 2 oder 3, **dadurch gekennzeichnet, dass** die mathematische Beziehung das Verhältnis zwischen dem ersten elektrischen Potentialwert und dem zweiten elektrischen Potentialwert ist.

6. Verfahren nach einem der vorstehenden Ansprüche, **dadurch gekennzeichnet, dass** der Schritt des Messens und Vergleichens der elektrischen Leitfähigkeit bei verschiedenen bekannten Konzentrationen der Substanz oder des Substanzgemisches, dessen antioxidative Wirkung bewertet werden soll, wiederholt wird.

7. Verfahren nach einem der vorstehenden Ansprüche, **dadurch gekennzeichnet, dass** das Lösungsmittel aus einem Gemisch aus einem ersten Lösungsmittel, in dem der Wirkstoff bei der Bildung freier Radikale gelöst ist, und einem zweiten Lösungsmittel besteht, in dem die Substanz oder das Substanzgemisch, dessen antioxidative Wirkung bewertet werden soll, gelöst ist.

8. Verfahren nach einem der vorstehenden Ansprüche, **dadurch gekennzeichnet, dass** der Wirkstoff bei der Bildung freier Radikale eine Diazoverbindung ist; wobei die erste und die zweite Lösung auf einer Temperatur von mehr als 36 °C gehalten werden.

9. Verfahren nach Anspruch 8, **dadurch gekennzeichnet, dass** die Diazoverbindung 2,2'-Azo-bis-(2-Aminopropan)dichlorid (APAB) ist.

## Revendications

1. Procédé d'évaluation d'activité antioxydante d'une substance ou d'un mélange de substances ; ledit procédé étant **caractérisé en ce qu'**il comprend une étape de mesure et de comparaison de la conductivité électrique comprenant les opérations consistant à :
- détecter une première valeur de conductivité électrique et une première valeur de pH d'une première solution comprenant un solvant et une substance active dans la production de radicaux libres ;
- détecter une seconde valeur de conductivité électrique et une seconde valeur de pH d'une seconde solution comprenant ledit solvant, ladite substance active dans la production de radicaux libres et une substance ou un mélange de substances dont on veut évaluer l'activité antioxydante ;
**caractérisé par**
le calcul d'une relation mathématique entre ladite première valeur de conductivité électrique et ladite seconde valeur de conductivité électrique ;
ladite première solution et ladite seconde solution ayant la même température, le même volume de solvant, la même composition de solvant et la même concentration de substance active dans la production de radicaux libres.

2. Procédé selon la revendication 1, **caractérisé en ce qu'**il comprend une étape de mesure et de comparaison du potentiel électrique comprenant les opérations consistant à :
- détecter une première valeur de potentiel électrique de ladite première solution ;
- détecter une seconde valeur de potentiel électrique de ladite seconde solution ;
- calculer une relation mathématique entre ladite première valeur de potentiel électrique et ladite seconde valeur de potentiel électrique.

3. Procédé selon la revendication 2, **caractérisé en ce que** ladite première et ladite seconde valeur de potentiel électrique sont détectées au moyen d'un pH-mètre.

4. Procédé selon l'une quelconque des revendications précédentes, **caractérisé en ce que** ladite relation mathématique est le rapport entre la première valeur de conductivité électrique et la seconde valeur de conductivité électrique.

5. Procédé selon la revendication 2 ou 3, **caractérisé en ce que** la relation mathématique est le rapport entre la première valeur de potentiel électrique et la seconde valeur de potentiel électrique.

6. Procédé selon l'une des revendications précédentes, caractérisé en que l'étape de mesure et de comparaison de la conductivité électrique est répétée à différentes concentrations connues de la substance ou du mélange de substances dont on veut évaluer l'activité antioxydante.

7. Procédé selon l'une des revendications précédentes, **caractérisé en ce que** ledit solvant est composé d'un mélange d'un premier solvant, dans lequel est dissoute la substance active dans la production de radicaux libres, et d'un second solvant, dans lequel est dissout la substance ou le mélange de substances dont on veut évaluer l'activité antioxydante.

8. Procédé selon l'une des revendications précédentes, **caractérisé en ce que** ladite substance active dans la production de radicaux libres est un composé diazoïque ; lesdites première et seconde solutions étant maintenues à une température supérieure à 36 °C.

9. Procédé selon la revendication 8, **caractérisée en ce que** le composé diazoïque est le dichlorure de 2,2'-azo-bis (2-aminopropane) (APAB).
